# EUROPÄISCHE PATENTSCHRIFT

(11) **EP 1 235 796 B1**
(45) Veröffentlichungstag und Bekanntmachung des Hinweises auf die Patenterteilung: **31.05.2006**
(21) Anmeldenummer: 00987243.3
(22) Anmeldetag: 14.11.2000
(51) Int. Cl.: C07C 303/44

(54) **VERFAHREN ZUR GEWINNUNG UND REINIGUNG SUBSTITUIERTER BENZOLSULFONATE**
METHOD FOR PRODUCING AND PURIFYING SUBSTITUTED BENZENE SULFONATES
PROCEDE POUR PREPARER ET PURIFIER DES SULFONATES DE BENZENE SUBSTITUES

(30) Priorität: 25.11.1999 DE 19956862
(43) Veröffentlichungstag der Anmeldung: 04.09.2002
(73) Patentinhaber: SIEMENS AKTIENGESELLSCHAFT, 80333 München (DE)
(72) Erfinder: FORBERT, Rainald, 65439 Flörsheim (DE); DIEHL, Thomas, 65439 Flörsheim (DE)
(86) Internationale Anmeldenummer: PCT/EP2000/011223
(87) Internationale Veröffentlichungsnummer: WO 2001/038299

(56) Entgegenhaltungen:
- DE-A- 3 337 921
- DE-B- 1 131 662
- US-A- 2 847 459
- US-A- 4 321 214
- US-A- 4 690 785

## Beschreibung

Die Erfindung betrifft ein Verfahren zur Gewinnung und Reinigung substituierter Benzolsulfonate, wobei ein Gemisch, das mindestens ein substituiertes Benzolsulfonat oder mindestens eine korrespondierende substituierte Benzolsulfonsäure, sowie Schwefeltrioxid, Schwefelsäure und/oder Chlorwasserstoff in freier oder gebundener Form enthält, in einer wässerigen Phase mit einem Alkalihydroxid neutralisiert und in dieser wässerigen Phase gereinigt wird und wobei das mindestens eine substituierte Benzosulfonat anschließend aus dieser wässerigen Phase abgetrennt wird.

Substituierte Benzolsulfonate haben einen weiten Einsatzbereich. Alkylbenzolsulfonate mit mehr als 5 Kohlenstoffatomen in der Alkylgruppe sind die am häufigsten eingesetzten seifenfreien Tenside, da sie gut reinigende, emulgierende, schaumbildende und benetzende Eigenschaften besitzen. Lineare Alkylbenzolsulfonate sind biologisch gut abbaubar und sind deshalb wesentlicher Bestandteil von vielen Waschmitteln und anderen Reinigungsmitteln. Amidosäurephenylestersulfonate dienen als Bleichaktivatoren in Waschmitteln und anderen Reinigungsmitteln, die Bleichen enthalten. Diese Aktivatoren haben mehrere vorteilhafte Eigenschaften wie exzellente Bleichleistung bei minimaler Schädigung von Gewebefarben, gute Kompatibilität mit Waschmaschinen und ein gutes Geruchsprofil in der Wäsche.

Aus dem Stand der Technik sind viele unterschiedliche Synthesewege für substituierte Benzolsulfonate bekannt.

Ein Verfahren der eingangs genannten Artist aus der US 2 847 459 A bekannt, wobei die nach der Neutralisierung erhaltene Reaktionsmischung als slurry (Schlamm, Dickstoff) bezeichnet wird.

Aus der DE 33 37 921 A ist es bekannt, eine Acyloxibenzolsäure mit einer wässerigen Natronlauge in Wasser unter guter Durchmischung zusammen zu bringen.

Es ist ferner aus "Surfactants" in Ullmann's Encyclopedia of Industrial Chemistry, Vol. A25, p. 747 ÷ 817, Weinheim 1994, und der DE 1 131 662 B bekannt, oberflächenwirksame Alkylbenzolsulfonate in der Weise zu gewinnen, daß man Alkylbenzole mit einer Seitenkette von 8 bis 20 Kohlenstoffatomen mit Oleum sulfoniert, die überschüssige Schwefelsäure abtrennt und im Anschluß daran das Reaktionsprodukt mit Natronlauge neutralisiert. Die so erhaltenen Produkte enthalten jedoch nach der Neutralisation noch 10 bis 15% Fremdsalze, die im wesentlichen aus Natriumsulfat bestehen, das aus dem überschüssigen Sulfonierungsmittel herrührt, sowie aus einer geringen Menge an Natriumchlorid, das aus dem sich an die Neutralisation gegebenenfalls anschließenden Bleichprozeß mit Natriumhypochlorit stammt.

In bekannter Weise wird die Befreiung des Alkylbenzolsulfonates von diesen anorganischen Salzen durch Extraktion mit organischen Lösungsmitteln, wie Alkoholen oder auch chlorierten Kohlenwasserstoffen, durchgeführt. Diese Verfahren sind jedoch umständlich und führen nicht immer zu den gewünschten Erfolgen.

Aus "Surfactants" in Ullmann's Encyclopedia of Industrial Chemistry, Vol. A25, p. 747 ÷ 817, Weinheim 1994, ist ferner bekannt, die Sulfonierung mit reinem Schwefeltrioxid durchzuführen, wodurch der Anfall von Fremdsalzen bei der Neutralisation verringert wird. Das Sulfonierungsverfahren mit Schwefeltrioxid in vergaster oder flüssiger Form ist aber, apparativ betrachtet, kompliziert. Außerdem enthalten die Natriumalkylbenzolsulfonate aus diesen Verfahren weiterhin einen Anteil an Fremdsalzen (Natriumsulfat, Natriumchlorid) im Prozentbereich bezogen auf die aktive Substanz.

Es ist weiterhin bekannt, als Sulfonierungsmittel Chlorsulfonsäure zu verwenden. In diesem Falle bilden sich teilweise Alkylbenzolsulfochloride, die bei der Weiterverarbeitung zur Bildung von Natriumchlorid und Natriumsulfat als Nebenprodukten führen.

Es ist weiterhin bekannt, Natriumalkylbenzolsulfonate mit Natriumchlorid aus wässerigen Lösungen, die auch Natriumsulfat gelöst enthalten können, auszusalzen. Es hat sich jedoch gezeigt, daß man auf diese Weise erst nach längeren Absitzzeiten zu fremdsalzarmen Alkylbenzolsulfonaten gelangt, die aber stets noch einen bestimmten Gehalt an Natriumsulfat aufweisen. Diese Produkte sind daher nicht zur Herstellung von gegen Natriumsulfat empfindlichen kosmetischen Erzeugnissen und ebenfalls nicht für den Einsatz als Emulgatoren in großtechnischen Prozessen, die in der Emulsionsphase stattfinden, verwendbar.

Aus der DE 1 131 662 B ist ferner bekannt, daß man Alkylbenzolsulfonate mit einem Gemisch aussalzen kann, das neben Natriumchlorid und Natriumsulfat noch einen Anteil an Alkylpolysulfonaten enthält, und daß die Schichtentrennung zwischen wässeriger Phase und alkylbenzolsulfonatreicher Phase dadurch schneller erzielt wird, wonach die als wässerige Paste anfallenden fremdsalzarmen Alkylbenzolsulfonate in bekannter Weise von der Unterschicht abgetrennt werden können. Dieses Verfahren hat den Nachteil, daß sich die verwendeten Alkylpolysulfonate zum einen Teil als unerwünschte Bestandteile im Produkt wiederfinden und zum anderen Teil das Abwasser aus dem Prozeß belasten. Außerdem betragen die Absitzzeiten auch nach diesem Verfahren noch mehrere Stunden.

Es sind weiterhin unterschiedliche Wege zur Synthese von Amidosäurephenylestersulfonaten aus dem Stand der Technik bekannt, wie z. B. in WO 95/07882, WO 96/28417, EP 0 922 694 und EP 0 922 695 beschrieben. Aus WO 99/09004 ist ein Verfahren zur Herstellung und Reinigung von Amidosäurephenylestersulfonaten bekannt.

Alle diese oben beschriebenen Verfahren zur Gewinnung und Reinigung von substituierten Benzolsulfonaten, insbesondere von p-substituierten Benzolsulfonaten, besitzen die gemeinsame Problematik, daß die substituierten Benzolsulfonate nach Durchführung der beschriebenen Verfahren in Gegenwart von Wasser und/oder gegebenenfalls von Lösungsmittel in pastösen bis gelartigen Konsistenzen vorliegen, so daß die substituierten Benzolsulfonate nur sehr langsam und mit unbefriedigender Reinheit aus den Synthese- und/oder Reinigungsgemischen abgetrennt werden können. Die Folge sind unwirtschaftlich große Trennapparate und unerwünscht hohe Produktverunreinigungen durch Nebenprodukte, wie z. B. Fremdsalze, oder Ausbeuteverluste beim Einsatz von Wäschen und/oder Bleichmitteln ["Surfactants" in Ullmann's Encyclopedia of Industrial Chemistry, Vol. A25, p. 747 ÷ 817, Weinheim 1994, und DE 1 131 662 B].

Der Erfindung lag daher die Aufgabe zugrunde, ein Verfahren zur Gewinnung und Reinigung von substituierten Benzolsulfonaten zu entwickeln, das zum einen nicht den Nachteil der sehr langsamen mechanischen Abtrennung des Produktes besitzt und zum anderen befriedigend hohe Reinheiten erzielt.

Diese Aufgabe wird erfindungsgemäß gelöst durch ein Verfahren zur Gewinnung und Reinigung substituierter Benzolsulfonate, wobei ein Gemisch, das mindestens ein substituiertes Benzolsulfonat oder mindestens eine korrepondierende substituierte Benzolsulfonsäure, sowie Schwefeltrioxid, Schwefelsäure und/oder Chlorwasserstoff in freier oder gebundener Form enthält, in einer wässerigen Phase mit einem Alkalihydroxid neutralisiert und in dieser wässerigen Phase gereinigt wird und wobei das mindestens eine substituierte Benzolsulfonat anschließend aus dieser wässerigen Phase abgetrennt wird, dadurch gekennzeichnet, daß das besagte Gemisch vor dem Eintrag in diese wässerige Phase in Partikel zerteilt wird, deren Sauterdurchmesser einen Wert aus dem Bereich von 1 µm bis 2 cm hat.

"Neutralisation" und damit sinnverwandte Wörter wie z. B. "neutralisieren" beziehen sich im Sinne dieser Erfindung ganz allgemein auf die Neutralisationsreaktion : Base + Säure → Salz + Wasser, und nicht unbedingt auf die speziellere Definition des Neutralstellens, die mit einem pH-Wert von ca. 7 verbunden wird.

Partikelschwärme bestehen in aller Regel nicht aus einheitlichen Partikeln gleicher Größe. Zur Charakterisierung der Partikelgröße eines Partikelschwarms wird in dieser Erfindung der Sauterdurchmesser verwendet, da er für verfahrenstechnische Trennoperationen mit umströmten Teilchen, wie z. B. die Filtration, ausschlaggebend ist. Der Sauterdurchmesser eines Partikelschwarms ist zu berechnen aus dem Volumen VP_{P} und der Oberfläche A aller Partikel eines Schwarms als: 6V_{P}/A, siehe M. Zogg, "Einführung in die mechanische Verfahrenstechnik", S. 15 ÷ 17, Stuttgart 1993.

Die substituierten Benzolsulfonate im Sinne dieser Erfindung besitzen die allgemeine Strukturformel : 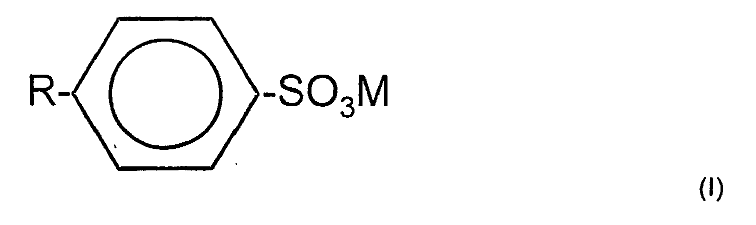 oder 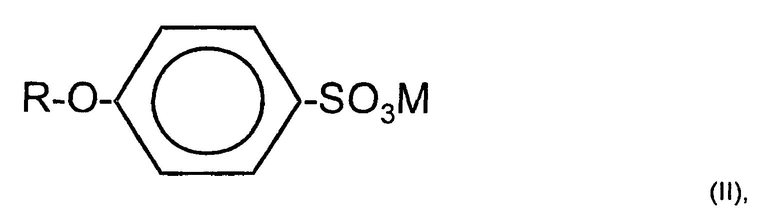 wobei die beiden Substituenten am Benzolring sowohl in der gezeigten para-Stellung als auch in meta- oder ortho-Stellung vorliegen können. Bevorzugt liegen die beiden Substituenten am Benzolring in der gezeigten para-Stellung vor. R ist eine lineare oder verzweigte Alkyl- oder Alkylengruppe mit 3 bis 20 Kohlenstoffatomen, die zum einen durch mindestens eine -O- und/oder -NH-Gruppe unterbrochen sein kann und die zum anderen durch mindestens eine Oxo-Gruppe substituiert sein kann. M steht für ein Alkalimetall, bevorzugt Natrium.

Besonders bevorzugte substituierte Benzolsulfonate sind lineare NatriumAlkylbenzolsulfonate mit 8 bis 16 Kohlenstoffatomen in der Alkylgruppe, sowie 4-Sulfophenyl-[(1-oxyalkanoyl)amino]alkanoate.

Ein ganz besonders bevorzugtes substituiertes Benzolsulfonat ist Natrium-n-Nonanoylamidohexanoyl-oxy-benzolsulfonat.

Eine besondere Ausführungsform ist dadurch gekennzeichnet, daß das Gemisch in Partikel zerteilt wird, deren Sauterdurchmesser einen Wert aus dem Bereich von 10 µm bis 0,5 cm, bevorzugt von 20 µm bis 1000 µm und besonders bevorzugt von 25 µm bis 300 µm, ganz besonders bevorzugt von 30 µm bis 100 µm hat.

Das Gemisch enthält bevorzugt 0,1 bis 20 %, besonders bevorzugt 1 bis 15 %, ganz besonders bevorzugt 2 bis 10 % Schwefeltrioxid, Schwefelsäure und/oder Chlorwasserstoff in freier oder gebundener Form.

Das Gemisch kann beliebige weitere Komponenten wie Nebenprodukte, Lösungsmittel und Katalysatoren enthalten.

Das Zerteilen des Gemischs in Partikel kann durch alle Prozesse durchgeführt werden, die dem Fachmann zu diesem Zweck bekannt sind, beispielsweise durch Zerstäuben, Zerwellen, Zertropfen oder Abtropfen, wie dies z. B. in P.Walzel, "Zerstäuben von Flüssigkeiten", Chem.-Ing.-Tech. 62 (1990) Nr. 12, S. 983 ÷ 994 beschrieben ist (im englischen Sprachgebrauch werden für diese Begriffe die Bezeichnungen "Jetting", "Sinusodial waves", "Laminar jet disintegration" und "Dripping" verwendet, s. "Spraying and Atomizing of Liquids" in Ullmann's encyclopedia of industrial chemistry, Vol. B2, p. 6-1 ÷ 6-14, Weinheim 1988). Bevorzugt wird das Gemisch vor dem Eintrag in die wässerige Phase oberhalb der Oberfläche dieser Phase zerstäubt, besonders bevorzugt durch Verwendung mindestens einer Einstoffdüse.

In einer besonderen Ausführungsform des Verfahrens wird das Gemisch vor dem Zerteilen mit Hilfe mindestens eines magnetischen Abscheiders und/oder mindestens eines Siebes und/oder mindestens eines Filters und/oder mindestens einer Naßzerkleinerungsmaschine und/oder mindestens einer Homogenisiermachine von größeren Partikeln befreit, die das Organ, daß das Gemisch in Partikel zerteilt, verstopfen können.

Durch Zugabe des Alkalihydroxids wird der pH-Wert der wässerigen Phase, in der das Gemisch neutralisiert und gereinigt wird, bevorzugt in einem Bereich von 7 bis 9, besonders bevorzugt von 7,5 bis 8,5 gehalten.

Durch direkte oder indirekte Kühlung wird die Temperatur der wässerigen Phase, in der das Gemisch neutralisiert und gereinigt wird, bevorzugt in einem Bereich von 0°C bis 80°C, besonders bevorzugt von 10°C bis 50°C, ganz besonders bevorzugt von 20°C bis 40°C gehalten.

In einer bevorzugten Ausführungsform des Verfahrens wird die wässerige Phase, in der das Gemisch neutralisiert und gereinigt wird, durch mindestens ein Rührorgan mit Umfangsgeschwindigkeiten aus dem Bereich von 0,05 bis 5 m/s, bevorzugt von 0,1 bis 1 m/s, besonders bevorzugt von 0,2 bis 0,5 m/s durchmischt.

Die Abtrennung des substituierten Benzolsulfonats aus der wässerigen Phase, in der das Gemisch neutralisiert und gereinigt wird, kann durch alle Prozesse durchgeführt werden, die dem Fachmann zu diesem Zweck bekannt sind, beispielsweise durch Sedimentation, Filtration oder Zentrifugation. Bevorzugt wird das substituierte Benzolsulfonat durch Vakuumfiltration aus der wässerigen Phase abgetrennt.

In einer besonderen Ausführungsform des Verfahrens wird ein Teil der abgetrennten wässerigen Phase zur Neutralisation und Reinigung des Gemischs wiederverwendet.

Während oder nach der Abtrennung des substituierten Benzolsulfonats aus der wässerigen Phase, in der das Gemisch neutralisiert und gereinigt wird, kann das substituierte Benzolsulfonat mit einem Waschsystem gewaschen werden. Bevorzugt besteht das Waschsystem zu mehr als 60 Gew.-% aus Wasser.

In einer besonderen Ausführungsform des Verfahrens wird das Waschsystem nach der Abtrennung von dem gewaschenen substituierten Benzolsulfonat ganz oder in Teilen als Waschsystem wiederverwendet und/oder der wässerigen Phase zur Neutralisation und Reinigung des Gemischs zugeführt.

Das substituierte Benzolsulfonat kann nach der Abtrennung aus der wässerigen Phase zur weiteren Aufreinigung getrocknet werden. Dazu sind alle Prozesse verwendbar, die dem Fachmann zu diesem Zweck bekannt sind.

Das Verfahren kann ganz oder in Teilen absatzweise oder kontinuierlich betrieben werden, vorzugsweise wird das Verfahren vollständig kontinuierlich betrieben.

Die Erfindung beruht unter anderem auf dem überraschenden Effekt, daß die Abtrennung eines substituierten Benzolsulfonats aus der wässerigen Phase sehr viel schneller und mit einem wesentlich besseren Trennergebnis, d. h. einer höheren Reinheit, verläuft, wenn das Gemisch, daß das substituierte Benzolsulfonat oder die korrespondierende substituierte Benzolsulfonsäure enthält, der wässerigen Phase zur Neutralisation und Reinigung in Form von Partikeln, die von ihrer Partikelgröße und -form her selber gut abtrennbar wären, zugeführt wurde, obwohl das zugeführte Gemisch selbst flüssig ist.

Die Vorteile des erfindungsgemäßen Verfahrens sind im wesentlichen darin zu sehen, daß ein substituiertes Benzolsulfonat mit hoher Reinheit und guter Ausbeute mit geringem apparativem und energetischem Aufwand gewonnen wird.

## Patentansprüche

1. Verfahren zur Gewinnung und Reinigung substituierter Benzolsulfonate, wobei ein Gemisch, das mindestens ein substituiertes Benzolsulfonat oder mindestens eine korrespondierende substituierte Benzolsulfonsäure, sowie Schwefeltrioxid, Schwefelsäure und/oder Chlorwasserstoff in freier oder gebundener Form enthält, in einer wässerigen Phase mit einem Alkalihydroxid neutralisiert und in dieser wässerigen Phase gereinigt wird und wobei das mindestens eine substituierte Benzolsulfonat anschließend aus dieser wässerigen Phase abgetrennt wird, **dadurch gekennzeichnet, daß** das besagte Gemisch vor dem Eintrag in diese wässerige Phase in Partikel zerteilt wird, deren Sauterdurchmesser einen Wert aus dem Bereich von 1 µm bis 2 cm hat.

2. Verfahren nach Anspruch 1, wobei das Gemisch in Partikel zerteilt wird, deren Sauterdurchmesser einen Wert aus dem Bereich von 10 µm bis 0,5 cm hat.

3. Verfahren nach Anspruch 1 oder 2, wobei das Gemisch 0,1 bis 20 % Schwefeltrioxid, Schwefelsäure und/oder-Chlorwasserstoff in freier oder gebundener Form enthält.

4. Verfahren nach mindestens einem der vorstehenden Ansprüche, wobei das Zerteilen durch Zerstäuben, Zerwellen, Zertropfen oder Abtropfen durchgeführt wird.

5. Verfahren nach mindestens einem der vorstehenden Ansprüche, wobei das Zerteilen durch Zerstäuben mittels einer Einstoffdüse durchgeführt wird.

6. Verfahren nach mindestens einem der vorstehenden Ansprüche, wobei das Gemisch vor dem Zerteilen mit Hilfe mindestens eines magnetischen Abscheiders und/oder mindestens eines Siebes und/oder mindestens eines Filters und/oder mindestens einer Naßzerkleinerungsmaschine und/oder mindestens einer Homogenisiermachine von größeren Partikeln befreit wird, die das Organ, daß das Gemisch in Partikel zerteilt, verstopfen können.

7. Verfahren nach mindestens einem der vorstehenden Ansprüche, wobei durch Zugabe des Alkalihydroxids der pH-Wert der wässerigen Phase, in der das Gemisch neutralisiert und gereinigt wird, in einem Bereich von 7 bis 9 gehalten wird.

8. Verfahren nach mindestens einem der vorstehenden Ansprüche, wobei durch direkte oder indirekte Kühlung die Temperatur der wässerigen Phase, in der das Gemisch neutralisiert und gereinigt wird, in einem Bereich von 0°C bis 80°C gehalten wird.

9. Verfahren nach mindestens einem der vorstehenden Ansprüche, wobei die wässerige Phase, in der das Gemisch neutralisiert und gereinigt wird, durch mindestens ein Rührorgan mit-Umfangsgeschwindigkeiten aus dem Bereich von 0,05 bis 5 m/s durchmischt wird.

10. Verfahren nach mindestens einem der vorstehenden Ansprüche, wobei die Abtrennung des substituierten Benzolsulfonats aus der wässerigen Phase, in der das Gemisch neutralisiert und gereinigt wird, durch Sedimentation. Filtration oder Zentrifugation, bevorzugt durch Vakuumfiltration aus der Wässerigen Phase erfolgt.

11. Verfahren nach mindestens einem der vorstehenden Ansprüche, wobei ein Teil der abgetrennten wässerigen Phase zur Neutralisation und Reinigung des Gemischs wiederverwendet wird.

12. Verfahren nach mindestens einem der vorstehenden Ansprüche, wobei während oder nach der Abtrennung des substituierten Benzolsulfonats aus der wässerigen Phase, in der das Gemisch neutralisiert und gereinigt wird, das substituierte Benzolsulfonat mit einem Waschsystem gewaschen wird.

13. Verfahren nach Anspruch 12, wobei das Waschsystem mindestens 60 Gew.-% Wasser enthält.

14. Verfahren nach Anspruch 12 oder 13, wobei das Waschsystem nach der Abtrennung von dem gewaschenen substituierten Benzolsulfonat ganz oder in Teilen als Waschsystem wiederverwendet und/oder der wässerigen Phase zur Teilen als Waschsystem wiederverwendet und/oder der wässerigen Phase zur Neutralisation und Reinigung des Gemischs zugeführt wird.

15. Verfahren nach mindestens einem der vorstehenden Ansprüche, wobei das substituierte Benzolsulfonat nach der Abtrennung aus der wässerigen Phase zur weiteren Aufreinigung getrocknet wird.

## Claims

1. Method for obtaining and purifying substituted benzene sulphonates wherein a mixture containing at least one substituted benzene sulphonate or at least one corresponding substituted benzene sulphonic acid as well as sulphur trioxide, sulphuric acid and/or hydrogen chloride in free or bound form is neutralized in an aqueous phase with an alkali hydroxide and is purified in this aqueous phase, the one or more substituted benzene sulphonates then being separated from this aqueous phase, **characterized in that** said mixture is broken down into particles having a Sauter diameter value ranging from 1 µm to 2 cm before being introduced into this aqueous phase.

2. Method according to Claim 1, wherein the mixture is broken down into particles having a Sauter diameter value ranging from 10 µm to 0.5 cm.

3. Method according to Claim 1 or 2, wherein the mixture contains 0.1 to 20% sulphur trioxide, sulphuric acid and/or hydrogen chloride in free or bound form.

4. Method according to at least one of the preceding Claims, wherein the breaking down is performed by jetting, sinusoidal waves, laminar jet disintegration or dripping.

5. Method according to at least one of the preceding Claims, wherein the breaking down is performed by atomization by means of a single-component nozzle.

6. Method according to at least one of the preceding Claims, wherein prior to the mixture being broken down, at least one magnetic separator and/or at least one screen and/or at least one filter and/or at least one wet comminution machine and/or at least one homogenizing machine is used to rid the mixture of larger particles which can block the means of breaking down the mixture into particles.

7. Method according to at least one of the preceding Claims, wherein the pH value of the aqueous phase in which the mixture is neutralized and purified is maintained in the range 7 to 9 by the addition of the alkali hydroxide.

8. Method according to at least one of the preceding Claims, wherein the aqueous phase in which the mixture is neutralized and purified is maintained in the range 0°C to 80°C by means of direct or indirect cooling.

9. Method according to at least one of the preceding Claims, wherein the aqueous phase in which the mixture is neutralized and purified is thoroughly mixed by at least one stirring element with circumferential speeds in the range 0.05 to 5 m/s.

10. Method according to at least one of the preceding Claims, wherein the separation of the substituted benzene sulphonate from the aqueous phase in which the mixture is neutralized and purified is performed by sedimentation, filtration or centrifugation, preferably by vacuum filtration.

11. Method according to at least one of the preceding Claims, wherein part of the separated aqueous phase is reused for neutralizing and purifying the mixture.

12. Method according to at least one of the preceding Claims, wherein during or after separation of the substituted benzene sulphonate from the aqueous phase in which the mixture is neutralized and purified, the substituted benzene sulphonate is washed using a washing system.

13. Method according to Claim 12, wherein the washing system contains at least 60% by weight water.

14. Method according to Claim 12 or 13, wherein the washing system, after separation from the washed substituted benzene sulphonate, is reused in whole or in part as a washing system and/or is fed to the aqueous phase for neutralizing and purifying the mixture.

15. Method according to at least one of the preceding Claims, whereby the substituted benzene sulphonate is dried for further purification following separation from the aqueous phase.

## Revendications

1. Procédé de préparation et de purification de benzènesulfonates substitués, dans lequel on neutralise dans une phase aqueuse par un hydroxyde de métal alcalin et on purifie dans cette phase aqueuse un mélange qui contient au moins un benzènesulfonate substitué ou au moins un acide benzènesulfonique substitué correspondant, ainsi que du trioxyde de soufre, de l'acide sulfurique et/ou du chlorure d'hydrogène sous forme libre ou liée, et dans lequel on sépare ensuite le au moins un benzènesulfonate substitué de cette phase aqueuse, **caractérisé en ce que** l'on fragmente ledit mélange avant l'introduction dans cette phase aqueuse en particules, dont le diamètre de Sauter a une valeur de l'ordre de 1 µm à 2 cm.

2. Procédé suivant la revendication 1, dans lequel on fragmente le mélange en particules, dont le diamètre de Sauter a une valeur de l'ordre de 10 µm à 0,5 cm.

3. Procédé suivant la revendication 1 ou 2, dans lequel le mélange contient de 0,1 à 20 % de trioxyde de soufre, d'acide sulfurique et/ou de chlorure d'hydrogène sous forme libre ou liée.

4. Procédé suivant au moins l'une des revendications précédentes, dans lequel on effectue la fragmentation par pulvérisation, par application d'ondes, par séparation sous forme de gouttes ou par égouttage.

5. Procédé suivant au moins l'une des revendications précédentes, dans lequel on effectue la fragmentation par pulvérisation au moyen d'une buse à une seule substance.

6. Procédé suivant au moins l'une des revendications précédentes, dans lequel on débarrasse le mélange avant la fragmentation, à l'aide d'au moins un séparateur magnétique et/ou d'au moins un tamis et/ou d'au moins un filtre et/ou d'au moins une machine de fragmentation à l'état humide et/ou d'au moins une machine d'homogénéisation, de particules assez grandes qui pourraient boucher l'organe qui fragmente le mélange en particules.

7. Procédé suivant au moins l'une des revendications précédentes, dans lequel on maintient, par addition de l'hydroxyde de métal alcalin, le pH de la phase aqueuse dans lequel on neutralise et on purifie le mélange dans le domaine de 7 à 9.

8. Procédé suivant au moins l'une des revendications précédentes, dans lequel on maintient par refroidissement direct ou indirect la température de la phase aqueuse dans laquelle on neutralise et on purifie le mélange dans un intervalle de 0°C à 80°C.

9. Procédé suivant au moins l'une des revendications précédentes, dans lequel on mélange la phase aqueuse, dans laquelle on neutralise et on purifie le mélange par au moins un organe d'agitation ayant des vitesses périphériques de l'ordre de 0,05 à 5 m/s.

10. Procédé suivant au moins l'une des revendications précédentes, dans lequel on effectue la séparation du benzènesulfonate substitué de la phase aqueuse, dans laquelle on neutralise et on purifie le mélange par sédimentation, par filtration ou par centrifugation, de préférence par filtration sous vide hors de la phase aqueuse.

11. Procédé suivant au moins l'une des revendications précédentes, dans lequel on réutilise une partie de la phase aqueuse séparée pour la neutralisation et la purification du mélange.

12. Procédé suivant au moins l'une des revendications précédentes, dans lequel on lave pendant ou après la séparation du benzènesulfonate substitué de la phase aqueuse dans laquelle on neutralise et on purifie le mélange, le benzènesulfonate étant substitué par un système de lavage.

13. Procédé suivant la revendication 12, dans lequel le système de lavage contient au moins 60 % en poids d'eau.

14. Procédé suivant la revendication 12 ou 13, dans lequel on réutilise en tout ou partie comme système de lavage le système de lavage après la séparation du benzènesulfonate substitué lavé et/ou on réutilise comme système de lavage en tout ou partie la phase aqueuse et/ou on envoie la phase aqueuse pour la neutralisation et la purification du mélange.

15. Procédé suivant au moins l'une des revendications précédentes, dans lequel on sèche le benzènesulfonate substitué après la séparation de la phase aqueuse pour le purifier davantage.
